# NEW EUROPEAN PATENT SPECIFICATION

(11) **EP 2 575 830 B2**
(45) Date of publication and mention of the opposition decision: **07.02.2024**
(45) Mention of the grant of the patent: 16.08.2017
(21) Application number: 11725215.5
(22) Date of filing: 01.06.2011
(51) Int. Cl.: A23L 29/256, A61K 35/20, A61P 1/02

(54) **PRE-THICKENED COMPACT LIQUID NUTRITIONAL COMPOSITION FOR DYSPHAGIA PATIENTS**
VORVERDICKTE KOMPAKTE FLÜSSIGE NAHRUNGSZUSAMMENSETZUNG FÜR PATIENTEN MIT DYSPHAGIE
COMPOSITION NUTRITIONNELLE LIQUIDE COMPACTE PRÉ-ÉPAISSIE POUR LES PATIENTS SOUFFRANT DE DYSPHAGIE

(30) Priority: 04.06.2010 WO PCT/NL2010/050342
(43) Date of publication of application: 10.04.2013
(73) Proprietor: N.V. Nutricia, 2712 HM Zoetermeer (NL)
(72) Inventor: SLIWINSKI, Edward Lucian, NL-5342 EM Oss (NL); DE KORT, Esther Jacqueline Petra, NL-6708 SE Wageningen (NL); VAN RAVESTEIJN, Christiaan Thedoor, NL-3037 GC Rotterdam (NL); LE FUR, Audrey Claire Lilie, F-29860 Bourg-Blanc (FR)
(74) Representative: Nederlandsch Octrooibureau
(86) International application number: PCT/NL2011/050390
(87) International publication number: WO 2011/152726

(56) References cited:
- WO-A1-03/055334
- WO-A1-2009/072886
- SPAGNUOLO P A ET AL: "Kappa-carrageenan interactions in systems containing casein micelles and polysaccharide stabilizers", FOOD HYDROCOLLOIDS, ELSEVIER, vol. 19, no. 3, 1 May 2005 (2005-05-01), pages 371-377, XP004719663, ISSN: 0268-005X, DOI: DOI:10.1016/J.FOODHYD.2004.10.003
- S. Thaiudom ET AL: "Effect of [kappa]-carrageenan on milk protein polysaccharide mixtures", International Dairy Journal, vol. 13, no. 9, 1 January 2003 (2003-01-01), pages 763-771, XP055191377, ISSN: 0958-6946, DOI: 10.1016/S0958-6946(03)00097-9
- J.M. GARCIA et al.: "Viscosity Measurements of Nectar- and Honey-thick Liquids: Product, Liquid, and Time Comparisons", Dysphagia, vol. 20, 2005, pages 325-335,
- STROWD, L. et al.: "Dysphagia Dietary Guidelines and the Rheology of Nutritional Feeds and Barium Test Feeds", Chest, vol. 133, no. 6, June 2008 (2008-06), pages 1397-1401,

## Description

### FIELD OF THE INVENTION

The present invention relates to a pourable liquid composition that comprises in a compact way a protein source, fat, digestible carbohydrates, dietary fibers and micronutrients.

### BACKGROUND OF THE INVENTION

Dysphagia patients have a swallowing problem. These patients often drink a liquid with an increased viscosity that may vary depending on the cause of their swallowing problem. The most prominent causes of dysphagia are Stroke, Parkinson's, Alzheimer's, Brain Damage and Multiple Sclerosis. These patient groups all need thickened drinks to be able to swallow their food. Dysphagia patients have a loss of sense in the oral cavity and lack a proper swallowing reflex causing a low viscosity liquid food to drip into the bronchia. Thickened nutritional compositions can prevent this leaking into the trachea.

Often dysphagia patients are undernourished or malnourished. This is mainly caused by bad eating habits due to fear of chocking during swallowing. There is therefore a need of a good medical food that can provide in a small volume all necessary ingredients in an easy to swallow format. The medical food would preferably be a nutrient-dense composition that would provide all necessary nutritional ingredients in a small volume. Hospitalized patients need significant amounts of protein and micronutrients in order to improve their health status. Technical difficulties exist in producing a stable, in particular a nutritional liquid composition with a constant reliable viscosity, having a high content of proteins.

Therefore, the problem underlying the present invention is to provide a shelf-stable liquid enteral composition for providing nutrition, either as a supplement, or as a complete nutrition, comprising a high content of an intact protein, as major protein source, in the smallest volume of liquid, and which supports nutrition and well-being in the different dysphagia patient groups mentioned above.

However, concentrating of liquids also increases the chance of undesired interactions between ingredients, thus reducing stability, especially during heating and long-term storage. In the context of the invention, 'shelf-stable' is defined as having a stability of more than 6 months (on the shelf) under normal storage conditions, i.e. at an ambient temperature of between 18 and 25 °C, and at a standard atmospheric pressure. This is particularly important for the dysphagia patient since it needs to trust the constant stable viscosity properties of the product. Commercial shelf-stable liquid products that are currently available have intact protein levels of 6g protein and 150 kcal in 100ml or 10g protein and 150 kcal in 100ml.

In the art, a solution to increase both protein and caloric content without imparting the stability of the end product is found in replacing part of the total protein by peptides or free amino acids. However, this seriously decreases taste appreciation and therefore voluntary intake of the nutritional composition by the patient group. On the other hand, many concentrates like condensed milks suffer from an incomplete nutrient profile, too high lactose levels, sticky mouth-feel, high viscosity, extreme sweetness and a high osmotic value, which is not appreciated by the consumer and rapidly increases feelings of fullness and satiety after consumption. This decreases the compliance of the product and may also result in malnutrition or under-nutrition.

WO2009072885 discloses a high-energy and high-protein liquid nutrition enteral compositions that contain micellar casein and caseinate. The aim of the invention is to provide low viscosity products with a viscosity of less than 120mPa.s. Due to low viscosity, the disclosed products are not suitable for dysphagia patients.

WO2009150183 discloses methods for the preparation of micellar casein. The calcium level may be 32 to 40mg/g protein. Suitable calcium sequestrants for keeping the calcium levels within the preferred range include mixtures of tri-, di-, and monosodium citrate with citric acid; the potassium salts of citric acid; the sodium and potassium salts of food grade organic acids including lactic, acetic and oxalic acids; and the sodium or potassium salts of phosphates, trisodium citrate and citric acid.

WO 02/098242 A1 (Nestle) discloses a calorically dense liquid oral supplement (2.25 kcal/ml) based on a (60:40) soy protein isolate/caseinate mixture with a protein level of 9 g/100 ml (16 en%), 12,25 g/100 ml of fat (49 en%), and 19.7 g/100 ml of digestible carbohydrates (35 en%).

US 5,683,984 (Nestec S.A.) and the corresponding EP patent 0 686 396 BI teach to replace all of the caseinate in a medium energy nutritional formulation (1 kcal/ml) by native micellar casein to obtain a formulation essentially containing native micellar casein with a low viscosity and a thermal stability to withstand sterilization. It discloses a heat-treated composition containing a maximum of 7 vol% of native micellar casein.

WO03/055334 relates to a method of preparing adapted foods where the composition allows the facilitation of the acts of eating or drinking for dysphagia patients.

WO 2009/072886 relates to low-viscosity energy-dense formulas that are designed for the treatment of the small population of dysphagia patients that can actually use these low viscosity products. However, there is a large group of dysphagia patients which cannot take these products. There is also a need for a product that does not provide a sticky mouthfeel, is stable over extended storage times, and addresses amylase resistance and a short texture. There is thus a need for thickening agents other than starch, that could provide short texture and excellent mouthfeel to the product.

Spagnuolo et al. "Kappa-carrageenan interactions in systems containing casein micelles and polysaccharide stabilizers", FOOD HYDROCOLLOIDS, ELSEVIER, vol. 19, no. 3,1 May 2005 (2005-05-01), pages 371-377, XP004719663, investigates how carragenan can be effective at preventing casein micelle macroscopic phase separation from polysaccharides.

S. Thaidom et al. "Effect of [kappaj-carrageenan on milk protein, polysaccharides mixtures". International Dairy Journal, vol. 13, no. 9,1 January 2003 (2003-01-01), pages 763-771, XP055191377 studies the effect of carrageenan on phase separation between polysaccharides and milk proteins in solution.

### SUMMARY OF THE INVENTION

The inventors surprisingly found that it is possible to prepare a nutrient- dense liquid composition that has a stable viscosity and a long shelf life in order to make it a suitable product for dysphagia patients. In one aspect, the invention lies in the use of anionic or acidic dietary fibers in combination with micellar casein.

It was found by the inventors that the use of dietary fibers in the composition has three benefits: to add nutritional value, to increase viscosity, and most importantly; to bind calcium thereby limiting the need for citrate. To be able to produce a product with a long shelf life it is necessary to take action to prevent formation of calcium-citrate crystals. Because the amount of calcium is often set by the nutritional composition, it is the amount of citrate that has to be restricted. It was surprisingly found that certain dietary fibers can serve to that end.

The compositions according to the present invention have a texture specifically suited for dysphagia patients. This includes: the right viscosity and also the right dependence of the viscosity of the shear rate, the right mouthfeel (non-sticky), the right texture (relatively short), and amylase resistance (limited decrease of viscosity once in contact with human saliva), and an extended shelf life (no formation of calcium-citrate crystals),

A preferred embodiment is a liquid composition suitable for dysphagia patients comprising at least 50 wt% micellar casein based on the total protein content, digestible carbohydrates, fat, and anionic soluble fibers, wherein the micellar casein provides at least 8 en% of the total energy of the composition, and carrageenan is present between 0.015 and 0.25 wt% of the total composition, and the viscosity of the composition is between 250 and 1800 mPas at 50/second at 20°C, preferably between 250 and 1800 mPas, more preferably at least 300 mPas, and even more preferably at least 400 mPas.

Compositions according to the invention are preferably used for the treatment of dysphagia patients. The composition according to the invention is particularly suitable for oral feeding of dysphagia patients and for the treatment and prevention of malnourishment or undernourishment associated with dysphagia ("associated with" means that the person is a dysphagia patient). The compositions are thus qualified as "liquid enteral compositions". The prevention aspect includes reducing the risk of or occurrence of malnourishment or undernourishment in a dysphagia patient.

### EMBODIMENTS OF THE PRESENT INVENTION

In a first embodiment [embodiment 1], the invention pertains to a liquid enteral nutritional composition with an energy density between 1.0 and 4.0 kcal/ml, a viscosity between 250 and 1800 mPas measured at a shear rate of 50/second at 20°C, comprising digestible carbohydrates and fat, wherein the composition further comprises;
at least one of:
a1. between 8-20 g protein per 100 ml of the composition, where micellar casein comprises at least 50wt% of the total protein content of the composition,
a2. between 16-45 en% protein, where micellar casein comprises at least 50 % of the protein caloric content;
and said composition further comprising:
b. water-soluble anionic fibers capable of sequestering of calcium, and
c. carrageenan between 0.015 and 0.25 g per 100 ml of the composition,
for use in treating/preventing malnourishment or undernourishment associated with dysphagia, and/or preventing/treating dysphagia.

In a second embodiment [embodiment 2], the invention pertains to a liquid nutritional composition with an energy density between 1.0 and 4.0 kcal/ml, a viscosity between 250 and 1800 mPas measured at a shear rate of 50/second at 20°C, comprising digestible carbohydrates and fat, wherein the composition further comprises;
at least one of:
a1. between 9 - 18 g protein per 100 ml of the composition, where micellar casein comprises at least 50wt% of the total protein content of the composition,
a2. between 16 - 45 en% protein, where micellar casein comprises at least 50 % of the protein caloric content;
and said composition further comprising:
b. water-soluble anionic fibers capable of sequestering of calcium, and
c. carrageenan between 0.015 and 0.25 g per 100 ml of the composition.

The invention also pertains to embodiment 1 or 2, wherein the water content is less than 60 wt%, preferably between 50 and 60 wt% of the composition.

The invention also pertains to any one of the preceding embodiments, wherein the combined amount of micellar casein and caseinate in the composition is at least 95 weight% of the total protein.

The invention also pertains to any one of the preceding embodiments, wherein said anionic fibers are selected from the group consisting of gum Arabic, oligosaccharides of xanthan gum, oligosaccharides of gellan gum, oligosaccharides of pectin, gum tragacanth , oligosaccharides of karaya gum, soy bean polysaccharides, oligosaccharides of alginate, oligosaccharides of konjac gum, in which part of the anionic groups may optionally be esterified.

The invention also pertains to any one of the preceding embodiments, wherein said composition comprises between 8-20 g protein per 100 ml of the composition, where micellar casein comprises at least 50wt% of the total protein content of the composition.

The invention also pertains to any one of the preceding embodiments, wherein the composition is in the form of a unit dose having a volume between 10 ml and 250 ml, preferably between 50 and 150 ml.

The invention also pertains to any one of the preceding embodiments, wherein the composition contains less than 1 wt% starch.

### DETAILED DESCRIPTION OF THE INVENTION

There is a need of compact nutritional products for dysphagia patients with a stable viscosity. Dysphagia patients have problems with swallowing food. In particular liquid nutrition is a problem since bad swallowing can cause the entry of food into the lungs. Therefore the inventors believe that compact liquid nutrition with a high energy density between 180 - 400 kcal/100ml, and comprising all essential nutritional ingredients is beneficial since less liquid has to be swallowed, and thus less swallowing mistakes can be made resulting in a better nutritional status of the patient.

In addition to the issue of stability and swallowing, the composition according to the present invention also addresses mouthfeel, texture and amylase resistance, all linked to the appreciation of the product by the dysphagia patient:

### Mouthfeel:

For "normal" consumers stickiness is not relevant. It can be desirable or non-desirable depending on the product type. For dysphagia patients it is however crucial that the product is not too sticky. This because a product having a relatively high viscosity and being quite sticky will stick to the palate and remain for longer in the oral cavity, being more different to remove. A sticky product will also lead to formation of more residues, which will be more difficult to remove. It is therefore of key importance that the thickeners selected to create texture do not contribute to the stickiness of the product.

### Texture:

For normal consumers texture (short or long texture) is not really relevant. A specific texture can be desired depending on the type of product. For dysphagia patients it is however crucial that the product although pourable and drinkable has a relatively short texture. This is an important property to prevent long threads from forming making it difficult for the product to stop from flowing/running. This is an important prerequisite for dysphagia patients because for them it is preferential to swallow discrete boluses instead of long threads. To have a very runny product that forms quite stable threads is not beneficial for dysphagia patients, because it can stimulate that product ends up in the longs.

### Amylase resistance:

For normal consumers sensitivity of a product to amylase is not an issue. It even can be appreciated when a product melts in the mouth. For dysphagia patients it is essential that a bolus maintains its viscosity during the oral phase and the act of swallowing. A melting bolus on the other hand will have a viscosity that decreases in the mouth which leading to potential unsafe situations. It means that the enteral composition should be thickened in such a way that during consumption and oral manipulation the viscosity does not decrease significantly. This makes that some thickeners have to be excluded, of which the main one starch.

The inventors have found that such high-energy liquid nutritional product can preferably be made when the product has a water content of less than 60 wt%. The low water content is advantageous to the compositions according to the invention since it provides for the required stability of viscosity and maintains the dosage volume low, both of great importance to the dysphagia patients. In a preferred embodiment according to the inventions the composition has a water content is between 50 and 60 wt%, a high protein content as defined in the claims, wherein the protein preferably provides at least 16 en%, the composition further containing digestible carbohydrates, low viscosity dietary fibers (such as soluble and insoluble oligosaccharides), including at least 0.02 wt% carrageenan. The carrageenan aids in adjusting the viscosity (and texture) of the composition to levels acceptable to the dysphagia patient.

Dysphagia can impair oral feeding, causing malnutrition, dehydration, and/or aspiration. Thin fluids require excellent muscle control and accurate coordination and timing between the swallowing and the breathing system. Thickened fluids slow the act of swallowing and by doing so, enhance safe swallowing. The provision of thickened fluids is a prescription for individuals with dysphagia. By determining the cause and severity of the dysphagia, health care professionals can determine the fluid thickness safest for an individual to swallow. If the prescription is not followed, the individual may face serious health consequences. Stage-1 is also defined as mildly thick. This consistency can be described as follows: steady fast flow; pours quickly from a cup but slower than regular, unmodified fluids; effort required to take this thickness via a standard bore straw; a viscosity ranging from about 150 to about 850 mPa.s measured at 50/s at 20°C. Stage-2 is also defined as moderately thick. This consistency can be described as follows: slow flow; cohesive and pours slowly; possible to drink from a cup, although fluid flows very slowly; difficult to drink using a straw, even if using a wide bore straw; a viscosity ranging from about 800 to about 1800 mPa.s measured at 50/s at 20°C. The compositions according to the present invention are suited for both stages. Hence, in one embodiment, the levels of carrageenan are adjusted to result in a composition having a viscosity between 250 and 850 mPas (at 50 s⁻¹, 20 °C); in another embodiment, the carrageenan-containing composition has a viscosity between 800 - 1800 mPas (at 50 s⁻¹, 20 °C).

### Protein

In particular, a liquid enteral nutritional composition is provided in which all or a major part of the protein comprises native micellar casein.

Micellar casein, also referred to as native micellar casein, refers to casein in the form of micelles. It is a high quality milk protein and naturally occurring in milk in a concentration of about 2.6 g/100 ml (Dairy Science and Technology, Walstra et al., CRC Press, 2006, page 3). It is concentrated by a process that does not, or does not substantially denature the casein proteins and it is marketed as Micellar Casein Isolate (MCI). Fresh skim milk is subjected to a micro filtration process, in much the same process used to concentrate whey protein, to produce a pure, substantially non-degraded milk protein. The resulting material contains between 90 % and 95 %, preferably more than 95 % by weight of micellar casein, the rest mainly being whey protein and other non-protein nitrogen and other constituents, such as lactose and inorganic salts, in particular calcium phosphate. The casein micelles generally have a hydrodynamic radius of 40 to 400 nm, a molecular weight of 106 to 109 Dalton and a calcium : phosphorous weight ratio of 1.4 to 2.4. This is much smaller than normal casein or other proteins of a similar molecular weight. This feature makes it possible to prepare liquid compositions with a high content of protein is required in the compositions according to the present invention.

The casein micelles have a low intrinsic viscosity and the liquid compositions as described in the prior art comprising said MCI have a low viscosity making them easy to drink for patients that do not suffer from problems with swallowing or for tube feeding. However, as addressed before, this low viscosity often strived for in the art would render this protein source unsuitable for the treatment of dysphagia patients. In the context of the invention, additional measures are therefore to be taken. It is noted that further increasing the MCI content of mixing MCI with other proteins may yield increased viscosity levels, but it would simultaneously have a detrimental effect on the shelf stability requirement. The actions taken by the inventors in order to raise viscosity but maintain shelf stability of the (heat-treated) composition are discussed further below.

Within the context of this invention, it is understood that micellar casein may also be provided by other milk protein sources, such as, for instance, sources with essentially preserve the natural 80:20 ratio of casein to whey, such as Milk Protein Concentrate (MPC), which is a powder product usually prepared by ultrafiltration with an average protein content of about 80 weight%, or Milk Protein Isolate (MPI), a powder product usually prepared by precipitation with an average protein content of more than 85 weight%, and skimmed concentrated milk.

According to one embodiment of the present invention, a liquid enteral nutritional composition is provided comprising an amount of protein per 100 ml of the composition as defined in the claims, preferably 9 to 18 g/100 ml, more preferably 10 to 16 g/100 ml; at least 50wt%, more preferably at least 60 wt%, most preferably at least 70 wt% of said protein comprises micellar casein.

In one embodiment, it is preferred that micellar casein and caseinate provide for at least 90 wt%, more preferably at least 95 % of the protein content.

In one embodiment, it is preferred to maintain relatively low whey protein levels, preferably lower than 30 wt%, more preferably lower than 15 wt%, most preferably up to 5 % whey of the protein content.

According to an embodiment of the present invention, a liquid enteral nutritional composition is provided comprising at least 16 en% protein based on the total energy content of the composition. Preferably the energy provided by the protein is between 16 and 45 en%, wherein at least 50 %, more preferably at least 60 %, most preferably at least 70 % of the protein energy content is provided for by micellar casein. According to another embodiment of the present invention, the composition has an energy density of at least 0.50 kcal/ml, more preferably at least 1.0 kcal/ml, particularly at least 1.5 kcal/ml of composition. It is preferred that the composition has an energy density of less than 4.0 kcal/ml. According to an embodiment of the present invention, the composition has an energy content between 1.0 and 4.0 kcal/ml, even more preferably between 1.4 and 3.5 kcal/ml, most preferred between 1.5 and 3.0 kcal/ml. In the context of this application, the % of total energy is abbreviated as en%; en% is thus short for energy percentage and represents the relative amount that a constituent contributes to the total caloric value of the composition. The Atwater constants 9 and 4 kcal/ g dry weight are used for calculating the energy content of lipids and protein or digestible carbohydrates respectively. For nutritional fibre the energy density of 2 kcal per g dry weight is applied.

The composition according to the invention is designed to either supplement a diet or to provide complete nutritional support. Hence, the composition according to the invention may further comprise at least fat and/or carbohydrate and/or a source of vitamins and minerals and/or a source of indigestible carbohydrates. Preferably, the composition according the invention is a nutritionally complete composition.

The composition is preferably heat-treated, i.e. being sterilized and/or pasteurized, preferably sterilized, using conditions conventional to those technologies.

### Fat

In one embodiment the present liquid enteral nutritional composition further comprises fat. The amount of fat may range between 5 and 95 %, preferably between 10 - 70 %, more preferably between 20 to 40 %, relative to the total energy content of the composition.

With regard to the type of fat, a wide choice is possible, as long as the fat is of food quality. The fat may either be an animal fat or a vegetable fat or both. Although animal fats such as lard or butter have essentially equal caloric and nutritional values and can be used interchangeably, vegetable oils are highly preferred in the practice of the present invention due to their readily availability, ease of formulation, absence of cholesterol and lower concentrations of saturated fatty acids. In one embodiment, the present composition thus comprises rapeseed oil, corn oil and/or sunflower oil.

The fat may include a source of medium chain fatty acids, such as medium chain triglycerides (MCT; chain length of fatty acid in the range of 6 to 12 carbon atoms, preferably 8 to 10 carbon atoms), a source of long chain fatty acids, such as long chain triglycerides (LCT; chain length of fatty acid higher than 12 carbon atoms) and phospholipid-bound fatty acids such as phospholipid-bound EPA or DHA, or any combination of the two types of sources. MCTs are beneficial because they are easily absorbed and metabolized in a metabolically-stressed patient. Moreover, the use of MCTs will reduce the risk of nutrient malabsorption. LCT sources, such as canola oil, rapeseed oil, sunflower oil, soybean oil, olive oil, coconut oil, palm oil, linseed oil, marine oil or corn oil are beneficial because LCTs may modulate the immune response in the human body. In one specific embodiment, the fat comprises 30 to 60 weight% of animal, algal or fungal fat, 40 to 70 weight% of vegetable fat and optionally 0 to 20 weight% of MCTs based on total fat of the composition. The animal fat preferably comprises a low amount of milk fat, i.e. lower than 6 weight%, especially lower than 3 weight% based on total fat. In particular, a mixture of corn oil, egg oil, and/or canola oil and specific amounts of marine oil is used. Egg oils, fish oils and algal oils are a preferred source of non-vegetable fats. Especially for compositions that are to be consumed orally, in order to prevent formation of off-flavours and to decrease a fishy after-taste, it is recommended to select ingredients that are relatively low in docosahexanoic acid (DHA), i.e. less than 6 weight%, preferably less than 4 weight% based on total fat. Marine oils containing DHA are preferably present in the composition according to the invention in an amount lower than 25 weight%, preferably lower than 15 weight% based on total fat. On the other hand, inclusion of eicosapentanoic acid (EPA) is highly desirable for obtaining the maximum health effect. Therefore, in another embodiment, the amount of EPA may range between 4 weight% and 15 weight%, more preferably between 8 weight% and 13 weight% based on total fat. The weight ratio EPA:DHA is advantageously at least 6:4, for example between 2:1 and 10:1. In yet another embodiment, the amount of EPA is very low, such as 0.1 to 1 weight%, preferably 0.3 weight% or 0.6 weight%, based on total fat.

Also, the liquid nutritional composition according to the invention may beneficially comprise one or more emulsifier(s). Commonly known emulsifiers may be used and generally the emulsifier(s) contributes to the energy content of the fat in said composition.

### Calcium sequestrants

The inventors have also addressed the problem of designing a high-protein products showing stability over time. Phase separation i.e. the formation of layers and changes in viscosity during storage of the product are unwanted. A problem associated with the use of micellar casein in the production of liquid enteral nutritional compositions with high protein content is the formation of calcium-acid complexes, such as calcium citrate, resulting in salt precipitates. Thereto, calcium sequestrants, particularly citric acid or a salt thereof, is added to the composition to adjust the pH and also to adjust Ca-ion activity. A certain Ca-ion activity is beneficial to maintain a desired viscosity of the composition during processing of the composition, e.g. during pasteurisation and/or sterilisation. Calcium, originating from the micellar casein, tends to react with acid, in particular the citric acid, thus forming calcium citrate crystals, which precipitate when pH of the composition decreases over time, giving rise to unacceptable short shelf stability. Already at a pH of 6.9, the formation of Ca-citrate crystals is progressing. On the other hand, a certain Ca-ion activity is beneficial to maintain a desired viscosity of the composition during processing of the composition, e.g. during pasteurisation and/or sterilisation. In particular a certain Ca-ion activity is beneficial to prevent a viscosity increase during heating.

Preferably calcium ion activity is between 10 and 100 ppm, more preferably between 20 and 80 ppm. To prevent formation and precipitation of insoluble calcium salts with time, calcium sequestrants should however be dosed with care. It is preferred that the amount of calcium sequestrants, preferably citrate, is lower than 0.4 wt% citrate/citric acid, to yield a shelf life of 6 months, preferably below 0.35 wt% citrate/citric acid, to yield a shelf life of 9 months, and preferably below 0.3 wt%, to yield a shelf life of 12 months.

Surprisingly it was found that in addition or instead of the above sequestrants of calcium known in the art like citrate and phosphate also anionic (water-)soluble fibers can be used. The advantages of anionic soluble fibers are however two-fold: (i) binding calcium and therewith optimizing pH and ensuring a long shelf life, and (ii) increasing the viscosity of the composition. Moreover, its use is not accompanied from any calcium precipitation affecting shelf-life.

Anionic soluble fibers to be considered generally comprise acidic groups from the group consisting of glucuronic acid, including 4-O-methyl glucuronic acid, galacturonic acid and mannuronic acid. These fibers can for instance be selected from the non-exhaustive group consisting of: gum Arabic (contains glucuronic acid, and 4-O-methyl glucuronic acid), oligosaccharides of xanthan gum (glucuronic acid), oligosaccharides of gellan gum (glucuronic acid), oligosaccharides of pectin (galacturonic acid), gum tragacanth (galacturonic acid), oligosaccharides of karaya gum (galacturonic acid, glucuronic acid), soy bean polysaccharides galacturonic acid), oligosaccharides of alginate (mannuronic acid, guluronic acid), oligosaccharides of konjac gum (O-acetate substitutes). Part of the anionic groups may be esterified. Oligosaccharides have a degree of polymerisation DP between 2 and 250, preferably 3 - 100.

Additionally, to further increase viscosity also dietary fibers other than anionic soluble dietary fibers can be used, provided these do not adversely affect product stability. These are refered to as 'inert' dietary fibers, meaning that these increase viscosity without causing instabilities. Useful examples are cellulosics (including microcrystalline cellulose or MCC), starch (albeit less preferred because it is digestible and thus sensitive to amylase), resistant starch, oligosaccharides of guar gum, oligosaccharides of tara gum, oligosaccharides of locust bean gum, oligosaccharides of xyloglucan, oligosaccharides of curdlan, (oligosaccharides of) β-glucans, fructo-oligosaccharides, galacto-oligosaccharides.

The amount of starch is preferably lower than 5 wt%, more preferably less than 1 wt%, most preferably less than 0.5 wt%.

### Carrageenan

For the present products according to the invention the right balance between pourability and viscosity is needed. For a safe swallow of a stage-1 drink a composition is thus provided having a viscosity of around 450 mPas at 50/second at 20°C, preferably between 250 and 700, most preferably between 350 and 600 mPas at 50/second at 20°C.

However, in practice very often the consistency of thickened products for dysphagia patients is not determined in a laboratory, but empirically judged by eye. The product is for instance stirred gently by hand, and the drink is allowed to run off a fork or spoon. This involves viscosity testing at relatively low shear rates. This behavior at low shear rates corresponds well with the behavior at higher shear rates to allow people to test the consistency at home. It is hypothesized that in a patient's throat, the product should not continue to run, but should be able to stop flowing to prevent product from flowing into the air-pipe. Therefore, it is desired that the product has a quite short texture, which means that when pouring a drink the flow should not continue as a thread, but should stop in a discrete fashion. Additionally it is required that the product does not have a sticky mouthfeel. This because product sticking to the palate induces repeated swallows after the first swallow of the bolus which is potentially dangerous for dysphagia patients.

As explained above, the desire for a high density of ingredients makes it necessary to use casein as protein source. It was surprisingly found that when using casein in combination with (viscous) soluble fibers, the composition may become sticky and threads may be formed. These threads make it difficult to swallow and are therefore dangerous for dysphagia patients. Although not wishing to be tied down to any theory, these threads are mainly composed of protein, and the fibers are believed to induce formation of these threads.

The inventors surprisingly found that the combination of a high density of micellar casein and one or more viscous soluble fiber(s) selected from the group consisting of agar, gelatine and carrageenan, preferably carrageenan-fibers, between 0.015 and 0.25 wt%, more preferably between 0.02 and 0.1 wt%, even more preferably between 0.025 and 0.075 wt%, give a stable viscous network that does not exhibit sticky properties, and has a stable viscosity over time. Carrageenan was found to give the most prolonged storage stability of the product, even in the absence of starch. These observations are summarized in Table 1. It shows that a number of soluble, viscous gums induce phase separation in the product, and thus limit shelf life. In most cases the instability occurred within two weeks. In combination with starch, gelatin, agar, and carrageenan provided a stable product, while without starch only carrageenan could give a stable product. Here, 'stable' thus means that in shelf life the product does not show phase separation (aggregation, syneresis or precipitation).

Hence, in an embodiment, the composition of the present invention contains no or little amounts of digestible starch, preferably less than 5 wt%, more preferably less than 1 wt%, most preferably less than 0.5 wt% thereof, and the composition further contains carrageenan in the amounts specified in the preceding paragraph. The stability without the presence of starch is an important feature since starch renders a product disadvantageously sensitive for breakdown by amylase from the saliva of dysphagia patients, thus decreasing the stability (of the viscosity) of the product. Although all types of carrageenan can be used (lambda, kappa, iota) alone or in combination, the best suitable option was shown to be iota carrageenan or a combination of iota and kappa carrageenan.

**TABLE 1. Effect of viscous soluble fibers on stability of nutritional compositions according to the invention**

| **VISCOUS SOLUBLE FIBER** | Combination with starch* | Not stable | Stable |
|---|---|---|---|
| Carobe gum | Yes | Phase separation | |
| Guar gum | Yes | Phase separation | |
| Tara gum | Yes | Phase separation | |
| Xanthan gum | Yes | Phase separation | |
| Xyloglycan | Yes | Phase separation | |
| Agar | Yes | | Stable |
| Gelatin | Yes | | Stable |
| Carrageenan | Yes | | Stable |
| Carrageenan | No | | Stable |
| * Medium or highly cross-linked waxy, tapioca or potato starch | | | |

### Viscosity and osmolarity

In the context of this invention, the viscosity referred to can be measured in a rotational rheometer using a cone-plate geometry at 20 °C at a shear rate of 50/s.

In one embodiment of the present invention, the viscosity of the liquid enteral nutritional composition is at least about 400 mPa.s, preferably about 450 mPa.s for a stage 1 product. For a stage 2 product the viscosity is preferably at least 850 mPa.s, preferably at least 1000mPa.s, preferably about between 1000 and 1500mPa.s, even more preferably about 1200mPa.s.

In one embodiment of the present invention, the osmolarity of the composition is preferably lower than 900 mOsm/l, more preferably lower than 800 mOsm/l, most preferably lower than 700 mOsm/l.

In one embodiment of the present invention, the density of the composition ranges between 1.05 g/ml and 1.20 g/ml, especially between 1.10 g/ml and 1.18 g/ml.

### Dosage unit

The liquid enteral nutritional composition according to the invention may have the form of a complete food, i.e. it can meet all nutritional needs of the user. As such, it preferably contains 1200 to 2500 kcal per daily dosage. The daily dosage amounts are given with respect to a daily energy supply of 2000 kcal to a healthy adult having a body weight of 70 kg. For persons of different condition and different body weight, the levels should be adapted accordingly. It is understood that the average daily energy intake preferably is about 2000 kcal. The complete food can be in the form of multiple dosage units, e.g. from 4 (250 ml/unit) to 20 (50 ml/unit) per day for an energy supply of 2000 kcal/day using a liquid enteral nutritional composition according to the invention of 2.0 kcal/ml.

The liquid enteral nutritional composition can also be a food supplement, for example to be used in addition to a non-medical food. Preferably as a supplement, the liquid enteral nutritional composition contains per daily dosage less than 1500 kcal, in particular as a supplement, the liquid enteral nutritional composition contains 400 to 1000 kcal per daily dose. The food supplement can be in the form of multiple dosage units, e.g. from 2 (250 ml/unit) to 10 (50 ml/unit) per day for an energy supply of 1000 kcal/day using a liquid enteral nutritional composition according to the invention of 2.0 kcal/ml. Dosage units are defined as physically identifiable separate units, usually in packaged form. A tube-feed is not comprised in the term "dosage unit" in the context of the invention, given its continuous administration regime.

In one embodiment of the present invention, the composition according to the invention is packaged. The packaging may have any suitable form, for example a block-shaped carton, e.g. to be emptied with a straw ; a carton or plastic beaker with removable cover ; a small-sized bottle or cup for example for the 80 ml to 200 ml range, and small cups for example for the 10 ml to 30 ml range.

In one embodiment of the present invention, a unit dosage comprises any amount of the liquid enteral nutritional composition according to the invention between 10 ml and 250 ml, the end values of this range included, preferably any amount between 25 ml and 200 ml, the end values of this range included, more preferably any amount between 50 ml and 150 ml, the end values of this range included, most preferably about 125 ml.

For example, a person receiving 50 ml unit dosages can be given 10 unit dosages per day to provide nutritional support using a liquid enteral nutritional composition according to the invention of 2.0 kcal/ml. Alternatively a person receiving 125 ml unit dosages can be given 4 or 5 or 6 or 7 or 8 unit dosages per day to provide nutritional support using a liquid enteral nutritional composition according to the invention of 2.0 kcal/ml. Such small dosage units are preferred because of better compliance.

The invention also pertains to the composition according to the above embodiments for use in treating/preventing dysphagia, and/or treating/preventing malnourishment or undernourishment associated with dysphagia. The composition is orally administered to the dysphagia patient or a person highly susceptible of dysphagia, such as persons suffering from stroke, Parkinson's, Alzheimer's, Brain Damage and Multiple Sclerosis.

### EXAMPLES

The examples 1 - 6 are composition suitable for stage-1 dysphagia, and are listed as columns in Table 2. Examples 7 - 12 in Table 3 list the ingredients of stage-2 compositions.

**Table 2: stage-1 compositions**

| | | EXAMPLE 1 | EXAMPLE 2 | EXAMPLE 3 | EXAMPLE 4 | EXAMPLE 5 | EXAMPLE 6 |
|---|---|---|---|---|---|---|---|
| Component | | Amount per 100 ml of product | Amount per 100 ml of product | Amount per 100 ml of product | Amount per 100 ml of product | Amount per 100 ml of product | Amount per 100 ml of product |
| Energy | | 245 kcal | 245 kcal | 245 kcal | 245 kcal | 160 kcal | 240 kcal |
| Protein | | 16.0 En% | 16.0 En% | 16.0 En% | 16.0 En% | 40.0 En% | 25.0 En% |
| Protein | | 10 g | 10 g | 10 g | 10 g | 16 g | 15 g |
| | Skim concentrated milk | - | 4.07 g | 3.49 g | 3.49 g | - | - |
| | Potassium caseinate | - | 2.71 g | 2.11 g | 2.11 g | - | - |
| | Calcium caseinate | - | 1.66 g | - | - | - | - |
| | Sodium-caseinate | 3.3 g | - | - | - | 3.2 g | 1.0 g |
| | Milk Protein Isolate | | 1.25 g | 4.11 g | - | - | - |
| | Micellar Casein Isolate | 6.3 g | - | - | 4.11 g | 12.8 g | 14.0 g |
| | Native micellar caseine | 6.0 | 4.26 | 6.08 | 6.70 | 12.0 | 13.3.0 |
| | Caseinate Whey | 3.3 0.3 | 4.37 1.06 | 2.11 1.52 | 2.11 0.90 | 3.2 0.60 | 1.0 0.70 |
| | Fat | 35 En% | 35 En% | 35 En% | 35 En% | 30 En% | 30 En% |
| | Fat mainly comprising canola oil | 9.3 g | 9.3 g | 9.3 g | 9.3 g | 5.3 g | 9.3 g |
| | Carbohydrates | 49 En% | 49 En% | 49 En% | 49 En% | 30 En% | 40 En% |
| | maltodextrine (DE19) | 29.4 g | 29.4 g | 29.4 g | 29.4 g | 12 g | 24 g |
| Minerals | | 16 % of RDI | 16 % of RDI | 16 % of RDI | 16 % of RDI | 16 % of RDI | 16 % of RDI |
| Vitamins | | 16 % of RDI | 16 % of RDI | 16 % of RDI | 16 % of RDI | 16 % of RDI | 16% of RDI |
| Dietary fiber | | 3.2 g* | 3.2 g* | 3.2 g* | 3.2 g* | 3.2 g* | 3.2 g* |
| carrageenan | | 0.0325 mg | 0.0325 mg | 0.0325 mg | 0.0325 mg | 0.0325 mg | 0.0325 mg |
| Viscosity (mPa.s at 20°C at 100 s⁻¹) | | 450 mPa.s | 450 mPa.s | 450 mPa.s | 450 mPa.s | 550 mPa.s | 450 mPa.s |
| Density | | 1.15 g/ml | 1.15 g/ml | 1.15 g/ml | 1.15 g/ml | 1.12 g/ml | 1.15 g/ml |
| Unit dosage | | 125 ml | 125 ml | 125 ml | 125 ml | 125 ml | 125 ml |

**Table 3: stage-2 compositions**

| | | **EXAMPLE 7** | | | **EXAMPLE 8** | | **EXAMPLE 9** | | **EXAMPLE 10** | | **EXAMPLE 11** | | **EXAMPLE 12** | |
|---|---|---|---|---|---|---|---|---|---|---|---|---|---|---|
| **Component** | | **Amount per 100 ml of product** | | | **Amount per 100 ml of product** | | **Amount per 100 ml of product** | | **Amount per 100 ml of product** | | **Amount per 100 ml of product** | | **Amount per 100 ml of product** | |
| Energy | | 245 kcal | | | 245 kcal | | 245 kcal | | 245 kcal | | 160 kcal | | 240 kcal | |
| Protein | | 16.0 En% | | | 16.0 En% | | 16.0 En% | | 16.0 En% | | 40.0 En% | | 25.0 En% | |
| Protein | | 9.6g g | | | | 9.6 g | 9.6 g | | 9.6 g | | 16g | | 15g | |
| | Skim concentrated milk | | - | | | 4.07 g | | 3.49 g | | 3.49 g | | - | | - |
| | Potassium caseinate | | - | | | 2.71 g | | 2.11 g | | 2.11 g | | - | | - |
| | Calcium caseinate | | - | | | 1.66 g | | - | | - | | - | | - |
| | Sodium-caseinate | | 3.3 g | | | - | | - | | - | | 3.2 g | | 1.0 g |
| | Milk Protein Isolate | | - | | | 1.25 g | | 4.11 g | | - | | - | | - |
| | Micellar Casein Isolate | | 6.3 g | | - | | | - | | 4.11 g | | 12.8 g | | 14.0 g |
| | Native micellar caseine | 6.0 | | | 4.26 | | 6.08 | | 6.70 | | 12.0 | | 13.3.0 | |
| | Caseinate | 3.3 | | | 4.37 | | 2.11 | | 2.11 | | 3.2 | | 1.0 | |
| | Whey | 0.3 | | | 1.06 | | 1.52 | | 0.90 | | 0.60 | | 0.70 | |
| Fat | | | | 35 En% | 35 En% | | 35 En% | | 35 En% | | 30 En% | | 30 En% | |
| | Fat mainly comprising canola oil | | | 9.3 g | 9.3 g | | 9.3 g | | 9.3 g | | 5.3 g | | 9.3 g | |
| Carbohydrates | | | | 49 En% | 49 En% | | 49 En% | | 49 En% | | 30 En% | | 40 En% | |
| | maltodextrine (DE19) | | | 29.4 g | 29.4 g | | 29.4 g | | 29.4 g | | 12 g | | 24 g | |
| Minerals | | | | 16 % of RDI | 16 % of RDI | | 16 % of RDI | | 16 % of RDI | | 16 % of RDI | | 16 % of RDI | |
| Vitamins | | | | 16 % of RDI | 16 % of RDI | | 16 % of RDI | | 16 % of RDI | | 16 % of RDI | | 16 % of RDI | |
| Dietary fiber | | | | 4.0 g* | 3.2 g** | | 4.0 g* | | 3.2 g** | | 3.6 g* | | 3.2 g** | |
| carrageenan | | | | 0.0325 mg | 0.0325 mg | | 0.0325 mg | | 0.0325 mg | | 0.0325 mg | | 0.0325 mg | |

| | **EXAMPLE 7** | | **EXAMPLE 8** | | **EXAMPLE 9** | | **EXAMPLE 10** | | **EXAMPLE 11** | | **EXAMPLE 12** | | | |
|---|---|---|---|---|---|---|---|---|---|---|---|---|---|---|
| **Component** | **Amount per 100 ml of product** | | **Amount per 100 ml of product** | | **Amount per 100 ml of product** | | **Amount per 100 ml of product** | | **Amount per 100 ml of product** | | **Amount per 100 ml of product** | | | |
| Viscosity (mPa.s at 20°C at 100 s⁻¹) | | 1200 mPa.s | 1200 mPa.s | | 1200 mPa.s | | 1200 mPa.s | | 1200 mPa.s | | 1200 mPa.s | | | |
| Density | | 1.15 g/ml | 1.15 g/ml | | 1.15 g/ml | | 1.15 g/ml | | 1.12 g/ml | | 1.15 g/ml | | | |
| Unit dosage | | 125 ml | 125 ml | | 125 ml | | 125 ml | | 125 ml | | 125 ml | | | |
| *fiber blend A | | | | | | | | | | | | | | |
| | 1000 gram mix | | fibre | % of mix | | % of fibre | | type | | Type | | | | |
| Soy oligosaccharides | 144.55 | | 108.4 | 14.46 | | 12.4 | | Insoluble | | Galacturonic acid | | | | |
| Cellulosics | 70.05 | | 65.7 | 7.01 | | 7.5 | | Insoluble | | neutral | | | | |
| Fructo-oligosaccharides 1 | 230.34 | | 209.1 | 23.03 | | 24.0 | | Soluble | | Neutral | | | | |
| Fructo-oligosaccharides 2 | 385.28 | | 351.8 | 38.53 | | 40.3 | | Soluble | | Neutral | | | | |
| Resistant starch | 15.73 | | 6.8 | 1.57 | | 0.8 | | Soluble | | Neutral | | | | |
| Gum Arabic | 154.05 | | 130.9 | 15.41 | | 15.0 | | Soluble | | glucuronic acid, and 4-O-methyl glucuronic acid | | | | |
| | | 1000.00 | | 872.8 | | 100.0 | | 100.0 | | | | | | |
| **fiber blend B | | | | | | | | | | | | | | |
| | 1000 gram mix | | fibre | | % of mix | | % of fibre | | type | | Type | | | |
| Soy oligosaccharides | 248.16 | | 186.1 | | 24.82 | | 23.6 | | Insoluble | | Galacturonic acid | | | |
| Cellulosics | 71.25 | | 66.8 | | 7.13 | | 8.5 | | Insoluble | | neutral | | | |
| Fructo-oligosaccharides 1 | 159.71 | | 145.0 | | 15.97 | | 18.4 | | Soluble | | Neutral | | | |
| **fiber blend B | | | | | | | | | | | | | | |
| | 1000 gram mix | fibre | | % of mix | | % of fibre | | type | | Type | | | | |
| Fructo-oligosaccharides 2 | 223.59 | 204.2 | | 22.36 | | 25.9 | | Soluble | | Neutral | | | | |
| Resistant starch | 157.25 | 68.0 | | 15.73 | | 8.6 | | Soluble | | Neutral | | | | |
| Gum Arabic | 140.05 | 119.0 | | 14.01 | | 15.1 | | Soluble | | glucuronic acid, and 4-O-methyl glucuronic acid | | | | |
| | 1000.00 | 789.1 | | 100.0 | | 100.0 | | | | | | | | |

## Claims

1. A liquid enteral nutritional composition with an energy density between 1.0 and 4.0 kcal/ml, a viscosity between 250 and 1800 mPas measured at a shear rate of 50/second at 20°C, comprising digestible carbohydrates and fat, wherein the composition further comprises;
at least one of:
a1. between 8-20 g protein per 100 ml of the composition, where micellar casein comprises at least 50wt% of the total protein content of the composition,
a2. between 16-45 en% protein, where micellar casein comprises at least 50 % of the protein caloric content;
and said composition further comprising:
b. water-soluble anionic fibers capable of sequestering of calcium, and
c. carrageenan between 0.015 and 0.25 g per 100 ml of the composition,
for use in treating/preventing malnourishment or undernourishment associated with dysphagia, and/or preventing/treating dysphagia.

2. The composition for use according to claim 1, wherein said anionic fibers are selected from the group consisting of gum Arabic, oligosaccharides of xanthan gum, oligosaccharides of gellan gum, oligosaccharides of pectin, gum tragacanth, oligosaccharides of karaya gum, soy bean polysaccharides, oligosaccharides of alginate, oligosaccharides of konjac gum, in which part of the anionic groups may optionally be esterified.

3. The composition for use according to claim 1 or 2,wherein the subject at risk of dysphagia is suffering from stroke, Parkinson's, Alzheimer's, Brain Damage or Multiple Sclerosis.

4. A liquid enteral nutritional composition with an energy density between 1.0 and 4.0 kcal/ml, a viscosity between 250 and 1800 mPas measured at a shear rate of 50/second at 20°C, comprising digestible carbohydrates and fat, wherein the composition further comprises;
at least one of:
a1. between 9-18 g protein per 100 ml of the composition, where micellar casein comprises at least 50wt% of the total protein content of the composition,
a2. between 16-45 en% protein, where micellar casein comprises at least 50 % of the protein caloric content;
and said composition further comprising:
b. anionic fibers capable of sequestering of calcium, and
c. carrageenan between 0.015 and 0.25 g per 100 ml of the composition.

5. The composition according to any of the preceding claims, wherein the water content is less than 60 wt%, preferably between 50 and 60 wt% of the composition.

6. The composition according to any of the preceding claims, wherein the combined amount of micellar casein and caseinate is at least 95 weight% of the total protein.

7. The composition according to any of the preceding claims, wherein the composition contains less than 5 wt% starch.

8. The composition according to any of the preceding claims, wherein the unit dosage is between 10 ml and 250 ml, preferably between 50 and 150 ml.

9. The composition according to any of the preceding claims, wherein said composition comprises between 9 to 18 g protein per 100 ml of the composition, where micellar casein comprises at least 50wt% of the total protein content of the composition.

10. The composition according to any of the preceding claims, wherein at least 70 wt% of said protein is micellar casein.

11. The composition according to any of the preceding claims, wherein whey protein levels are lower than 15 wt% of the protein content.

## Patentansprüche

1. Flüssige enterale Nahrungszusammensetzung mit einer Energiedichte zwischen 1,0 und 4,0 kcal/ml, einer Viskosität zwischen 250 und 1800 mPas, gemessen bei einer Scherrate von 50/Sekunde bei 20°C, umfassend verdauliche Kohlenhydrate und Fett, wobei die Zusammensetzung ferner umfasst:
wenigsten eines von:
a1. zwischen 8-20 g Protein pro 100 ml der Zusammensetzung, wobei mizellares Casein wenigstens 50 Gew.-% des gesamten Proteingehalts der Zusammensetzung umfasst,
a2. zwischen 16-25 en% Protein, wobei mizellares Casein wenigstens 50% des Proteinkaloriengehalts umfasst;
und wobei die Zusammensetzung ferner umfasst:
b. wasserlösliche anionische Fasern, die Kalzium sequestrieren können, und
c. Carrageen zwischen 0,015 und 0,25 g pro 100 ml der Zusammensetzung,
zur Verwendung in Behandlung/Vermeidung von Mangelernährung oder Unterernährung, die mit Dysphagie verbunden ist, und/oder Vermeidung/Behandlung von Dysphagie.

2. Zusammensetzung zur Verwendung nach Anspruch 1, wobei die anionischen Fasern ausgewählt sind aus der Gruppe bestehend aus Gummi Arabikum, Oligosacchariden von Xanthangummi, Oligosacchariden von Gellangummi, Oligosacchariden von Pektin, Gummi Tragacanth, Oligosacchariden von Karayagummi, Sojabohnenpolysacchariden, Oligosacchariden von Alginat, Oligosacchariden von Konjakgummi, bei denen ein Teil der anionischen Gruppen optional verestert sein kann.

3. Zusammensetzung zur Verwendung nach Anspruch 1 oder 2, wobei die für Dysphagie gefährdete Person unter Schlaganfall, Parkinson, Alzheimer, Hirnschädigung oder Multiple Sklerose leidet.

4. Flüssige Nahrungszusammensetzung mit einer Energiedichte zwischen 1,0 und 4,0 kcal/ml, einer Viskosität zwischen 250 und 1800 mPas gemessen bei einer Scherrate von 50/Sekunde bei 20°C umfassend verdauliche Kohlenhydrate und Fett, wobei die Zusammensetzung ferner umfasst:
wenigstens eines von:
a1. zwischen 9 bis 18 g Protein pro 100 ml der Zusammensetzung, wobei mizellares Casein wenigstens 50 Gew.-% des gesamten Proteingehalts der Zusammensetzung umfasst,
a2. zwischen 16-45 en% Protein, wobei mizellares Casein wenigstens 50% des Proteinkaloriengehalts umfasst;
und wobei die Zusammensetzung ferner umfasst:
b. anionische Fasern, die Kalzium sequestrieren können, und
c. Carrageen zwischen 0,015 und 0.25 g pro 100 ml der Zusammensetzung.

5. Zusammensetzung nach einem der vorangehenden Ansprüche, wobei der Wassergehalt weniger als 60 Gew.-%, bevorzugt zwischen 50 und 60-Gew.-% der Zusammensetzung, ist.

6. Zusammensetzung nach einem der vorangehenden Ansprüche, wobei die kombinierte Menge an mizellarem Casein und Caseinat wenigstens 95 Gew.-% des gesamten Proteins ist.

7. Zusammensetzung nach einem der vorangehenden Ansprüche, wobei die Zusammensetzung weniger als 5 Gew.-% Stärke enthält.

8. Zusammensetzung nach einem der vorangehenden Ansprüche, wobei die Dosierungseinheit zwischen 10 ml und 250 ml, bevorzugt zwischen 50 und 150 ml, ist.

9. Zusammensetzung nach einem der vorangehenden Ansprüche, wobei die Zusammensetzung zwischen 9 bis 18 g Protein pro 100 ml der Zusammensetzung umfasst, wobei mizellares Casein wenigstens 50 Gew.-% des gesamten Proteingehalts der Zusammensetzung umfasst.

10. Zusammensetzung nach einem der vorangehenden Ansprüche, wobei wenigstens 70 Gew.-% des Proteins mizellares Casein ist.

11. Zusammensetzung nach einem der vorangehenden Ansprüche, wobei Molkeproteingehalte geringer als 15 Gew.-% des Proteingehalts sind.

## Revendications

1. Composition nutritionnelle entérale liquide avec une densité énergétique comprise entre 1,0 et 4,0 kcal/ml, une viscosité comprise entre 250 et 1 800 mPa mesurée à un taux de cisaillement de 50/seconde à 20°C, comprenant des hydrates de carbone digestibles et de la matière grasse, dans laquelle la composition comprend en outre ;
au moins un parmi :
a1. entre 8 et 20 g de protéines pour 100 ml de la composition, la caséine micellaire représentant au moins 50 % en poids de la teneur protéique totale de la composition,
a2. entre 16 et 45 % en énergie de protéines, la caséine micellaire représentant au moins 50 % de la teneur protéique calorique ;
et ladite composition comprenant en outre :
(b) des fibres anioniques hydrosolubles capables de séquestrer le calcium, et
(c) du carraghénane, entre 0,015 et 0,25 g pour 100 ml de composition,
à utiliser pour le traitement/la prévention de la malnutrition ou de la sous-alimentation associée à la dysphagie, et/ou la prévention/le traitement de la dysphagie

2. Composition à utiliser selon la revendication 1, dans laquelle lesdites fibres anioniques sont sélectionnées dans le groupe constitué par la gomme arabique, des oligosaccharides de gomme de xanthane, des oligosaccharides de gomme de gellane, des oligosaccharides de pectine, la gomme adragante, des oligosaccharides de gomme de karaya, des polysaccharides de graines de soja, des oligosaccharides d'alginate, des oligosaccharides de gomme de konjac, où une partie des groupes anioniques peut éventuellement être estérifiée.

3. Composition à utiliser selon la revendication 1 ou 2, dans laquelle ledit sujet présentant un risque de dysphagie est atteint d'accident vasculaire cérébral, de la maladie de Parkinson, de la maladie d'Alzheimer, de lésions cérébrales ou de la sclérose en plaques.

4. Composition nutritionnelle liquide avec une densité énergétique comprise entre 1,0 et 4,0 kcal/ml, une viscosité comprise entre 250 et 1 800 mPa mesurée à un taux de cisaillement de 50/seconde à 20°C, comprenant des hydrates de carbone digestibles et de la matière grasse, dans laquelle la composition comprend en outre ;
au moins un parmi :
a1. entre 9 et 18 g de protéines pour 100 ml de la composition, la caséine micellaire représentant au moins 50 % en poids de la teneur protéique totale de la composition,
a2. entre 16 et 45 % en énergie de protéines, la caséine micellaire représentant au moins 50 % de la teneur protéique calorique ;
et ladite composition comprenant en outre :
(b) des fibres anioniques capables de séquestrer le calcium, et
(c) du carraghénane, entre 0,015 et 0,25 g pour 100 ml de composition

5. Composition selon l'une quelconque des revendications précédentes, dans laquelle la teneur en eau est inférieure à 60 % en poids, de préférence entre 50 et 60 % en poids de la composition.

6. Composition selon l'une quelconque des revendications précédentes, dans laquelle dans lequel la quantité combinée de caséine micellaire et de caséinate est d'au moins 95 % en poids de la protéine totale.

7. Composition selon l'une quelconque des revendications précédentes, dans laquelle la composition contient moins de 5 % en poids d'amidon.

8. Composition selon l'une quelconque des revendications précédentes, dans laquelle la dose unitaire est comprise entre 10 ml et 250 ml, de préférence entre 50 et 150 ml.

9. Composition selon l'une quelconque des revendications précédentes, dans laquelle la composition comporte entre 9 et 18 g de protéines pour 100 ml de la composition, où la caséine micellaire représentant au moins 50 % en poids de la teneur protéique totale de la composition.

10. Composition selon l'une quelconque des revendications précédentes, dans laquelle au moins 70 % en poids desdites protéines sont de la caséine micellaire.

11. Composition selon l'une quelconque des revendications précédentes, dans laquelle des niveaux de protéines de lactosérum sont inférieurs à 15 % en poids de la teneur protéique.
